# EUROPÄISCHE PATENTSCHRIFT

(11) **EP 1 231 949 B1**
(45) Veröffentlichungstag und Bekanntmachung des Hinweises auf die Patenterteilung: **03.03.2004**
(21) Anmeldenummer: 00984834.2
(22) Anmeldetag: 12.10.2000
(51) Int. Cl.: A61L 27/38, A61L 27/60, C12N 5/00, C12N 5/06

(54) **PARTIKULÄRES KONSTRUKT ZUR VERWENDUNG IN DER TRANSPLANTATIONSMEDIZIN**
PARTICULATE CONSTRUCT FOR USE IN THE TRANSPLANTATION FIELD
STRUCTURE PARTICULAIRE A UTILISER DANS LE DOMAINE DE LA TRANSPLANTATION

(30) Priorität: 12.10.1999 DE 19949290
(43) Veröffentlichungstag der Anmeldung: 21.08.2002
(73) Patentinhaber: Medusa Beteiligungsverwaltungs-Gesellschaft Nr. 79 mbH, 60322 Frankfurt (DE)
(72) Erfinder: BETTERMANN, Albrecht, 20249 Hamburg (DE); BUCHHOLZ, Rainer, 14641 Nauen (DE); HÜBNER, Holger, 13351 Berlin (DE); SCHNEIDER, Claudia, 13509 Berlin (DE)
(74) Vertreter: Jungblut, Bernhard Jakob, Dr.
(86) Internationale Anmeldenummer: PCT/DE2000/003658
(87) Internationale Veröffentlichungsnummer: WO 2001/026701

(56) Entgegenhaltungen:
- EP-A- 0 213 908
- WO-A-99/15637
- DE-A- 4 438 015
- US-A- 5 830 507

## Beschreibung

Die Erfindung betrifft eine Verwendung eines biologisch aktiven partikulären Konstrukts auf Basis einer räumlichen Trägerstruktur aus zumindest einem ausgehärteten biokompatiblen Polymeren, wobei in der Trägerstruktur eine Mehrzahl von Zellen zumindest eines humanen Zelltyps eingebettet ist, ein für die Verwendung besonders geeignetes partikuläres Konstrukt sowie ein Verfahren zu dessen Herstellung.

In der Transplantationsmedizin, insbesondere zur Heilung von tiefen Hautdefekten, kommen verschiedene Ansätze zur Anwendung. Die Standardmethode ist die Transplantation von patienteneigenen (autologen) Hautarealen. Bei Patienten mit beispielsweise großflächigen Verbrennungen ist eine Versorgung der Wunden mit autologen Transplantaten aufgrund der der geringen Restfläche intakter Hautareale offensichtlich aus Kapazitätsgründen begrenzt. Die Nutzung von Fremdhauttransplantaten ist aufgrund regelmäßig eintretender immunologischer Abstoßungsreaktionen allenfalls als Zwischenlösung geeignet.

Ein weiterer Ansatz ist die in vitro Kultivierung von insbesondere autologen Hautzellen und deren Implantation als flächige Gewebekonstrukte. Zum Verständnis dieser Technik ist es hilfreich, die Histologie der Haut zu beschreiben. Die Haut besitzt drei Hauptschichten, die sich in ihrer Funktion unterscheiden. Ausgehend von der Oberfläche beginnt der Aufbau mit der Epidermis. Diese ist ein mehrschichtiges, verhorntes Plattenepithel, worin die Keratinozyten mit einem Anteil von ca. 75% überwiegen. Mitotische Vorgänge finden nur im Stratum basale, der untersten Epidermisschicht, statt. Sie ist fest mit der darunter befindlichen Basalmembran, einer extrazellulären Matrix, verbunden. Von dort ausgehend differenzieren die Zellen unter Veränderung ihrer Morphologie bis in das Stratum corneum, der äußersten Hautschicht, aus, wo sie dann nach einer gewissen Zeit abgestoßen werden, der natürliche Erneuerung der Haut. Unterhalb der Basalmembran liegt die aus Bindegewebe bestehende Dermis. Diese ist eine Kombination aus Fibroblasten, Interzellularsubstanzen und Fettzellen. Die Interzellularsubstanzen bestehen z.B. aus Kollagen, Fibronektin und Elastin. Die Dermis dient der Stabilisierung der Haut und reguliert den Hautdruck. In ihr differenzieren die Keratinozyten. Die Tela subcutanea schließt die Haut zu den darunter liegenden Strukturen, Faszien, Knochen und/oder Muskulatur, ab.

Einer Konzeption der Schaffung einer künstlichen Haut liegt die Technolgie der flächigen Gewebekonstrukte zugrunde. Diese haben eine Sandwichstruktur enthaltend ein flächiges Substrat und eine auf dem flächigen, nicht lebenden Substrat anhaftende Schicht von kultivierten, lebenden Körperoberflächenzellen, insbesondere Fibroblasten und/oder Keratinozyten. Hierbei sind die Körperoberflächenzellen nicht zwingend ein Zellverband, wie in einem Organismus vorkommend. Flächige Gewebekonstrukte werden zum Verschluß und zur Regeneration von Wunden, insbesondere Verbrennungswunden, verwendet. Flächige Gewebekonstrukte bekannten Aufbaus sind beispielsweise in den Literaturstellen US-A 5,131,907, US-A 5,273,900 (hierin ist eine umfängliche Hintergrunddarstellung der technologischen Zusammenhänge gegeben), US-A 5,282,859, US-A 5,800,537 und US-A 5,888,248 beschrieben. Diesem Stand der Technik ist gemeinsam, daß als Substrat beispielsweise Collagen verwendet wird und daß Körperoberflächenzellen, beispielsweise Fibroblasten und/oder Keratinozyten, darauf zu einem dichten Zellrasen kultiviert sind. Der Zellrasen ist also mehrschichtig und weist eine voll ausgebildete Kontaktinhibierung auf. Im Ergebnis wird ein vollständiges Gewebe (im eigentlichen Sinne des Ausdrucks) in vitro hergestellt und gleichsam kopfüber (Substrat außenliegen) in eine Wunde eingebracht. Solche flächigen Gewebekonstrukte haben mehrere Nachteile. Zum ersten ist ein Substrat beispielsweise aus Collagen, sei es unvernetzt oder vernetzt, nicht hinreichend elastisch bzw. nicht hinreichend elastisch dehnbar, um bei (dreidimensional) komplexen Wundenformen im gesamten Wundbereich anliegend eingebracht werden zu können. Selbst bei weniger komplexen Wundenformen ist die Anwendung aufwendig, da der Arzt ein vollständiges Anliegen bewirken und gewährleisten muß. Ein vollständiges Anliegen ist jedoch eine wesentliche Voraussetzung für ein Anwachsen der Zellen an das Gewebe des Patienten. Weiterhin ist ein beachtlicher Anteil der Zellen in einem dichten Zellrasen kontaktinhibiert mit der Folge, daß eine Proliferation und folglich ein Wachstum bzw. ein Anwachsen in nicht befriedigendem Maße stattfindet. Insgesamt zeigt es sich, daß die insofern bekannten Gewebekonstrukte nicht hinreichend sicher anwachsen, wodurch im Falle des Nichtanwachsens wiederholte Behandlungen mit der künstlichen Haut und/oder Transplantationen zwingend erforderlich werden. Die Anheilungsrate beträgt in der Praxis meist nicht mehr als 30%.

Weiterhin bekannt ist eine Technologie, bei welcher nicht kontaktinhibierte, wenig differenzierte Zellen in einem humanen Fibrinkleber suspendiert sind und diese Suspension auf die Wunde aufgesprüht wird. Fibrinkleber besteht aus zwei Komponenten, dem Fibrinogen sowie dem Thrombin, welches die enzymatische Aushärtung des Fibrinogen bewirkt. Die applikationsfertige Zellsuspension wird dadurch hergestellt, daß zunächst die Zellen in der Fibrinogen Komponente suspendiert werden und unmittelbar vor der Applikation das Thrombin zugegeben wird. Diese Technologie führt zwar zu einem verbesserten Anwachsen von intakten Zellen, weist jedoch andere Nachteile auf. Insbesondere nachteilig ist, daß die Zellen keinerlei Schutz gegen Scherbelastungen besitzen und bei der Applikation oder bei Bewegung im Wundenbereich mechanisch überbelastet werden können. Weiterhin ist der Pegel proliferationsfördernder Signalstoffe, insbesondere der Wachstumsfaktoren, im Bereich der aufgesprühten Zellen eher niedrig und die Wachstumsrate insofern verbesserungsfähig.

Partikuläre Konstrukte des eingangs genannten Aufbaus sind in anderen Zusammenhängen bekannt, nämlich der biotechnologischen Herstellung von Substanzen durch geeignete und immobilisierte Organismen. Die Literaturstelle US-A-4,647,536 beschreibt die Vorteile der Verwendung solcher Konstrukte enthaltend tierische oder pflanzliche Zellen in biotechnologischen Säulenreaktoren. Eine ausführlichere Darstellung dieser Technologie ist in der Literaturstelle Eur. J. Microbiol. Biotechnol. (1983) 17:319-326 gegeben.

Aus der Literaturstelle WO99/15637 sind partikuläre Konstrukte zur Verwendung u.a. in der Transplantationmedizin bekannt, welche aus einem massiven Fibrinkern und auf der Oberfläche des Kerns angeordneten humanen Zellen bestehen. Das Fibrin ist hochvernetzt. Nachteilig bei diesen Konstrukten ist, daß das Zellwachstum und folglich die Gewebebildung im wesentlichen zweidimensional erfolgt und der Pegel an Wachstumsfaktoren vergleichsweise niedrig ist. Zudem sind die Zellen nicht gegen Scherbelastungen geschützt aufgrund ihrer außenseitigen Anordnung. Dies ist - neben diesbezüglicher Nachteile beim Einbringen in eine Wunde - bereits bei der Herstellung und Aufbereitung der Konstrukte nachteilig. Schließlich neigen solche Kostrukte zum Verklumpen.

Der Erfindung liegt das technische Problem zugrunde, Zellen enthaltende Konstrukte für die Transplantationsmedizin anzugeben, welche eine zügige und möglichst vollständige Rekonstruktion geschädigter Hautareale gewährleisten und gleichzeitig in der Applikation einfach sind.

Zur Lösung dieses technischen Problems lehrt die Erfindung die Verwendung eines biologisch aktiven partikulären Konstrukts auf Basis einer räumlichen Trägerstruktur aus zumindest einem ausgehärteten biokompatiblen Polymeren, wobei in der Trägerstruktur eine Mehrzahl von Zellen zumindest eines (vorzugsweise humanen) Zelltyps eingebettet ist, in der Transplantationsmedizin.

Mit der Erfindung werden mehrere Vorteile in Kombination miteinander erzielt. Zum ersten ist die Konzentration von Wachstumsfaktoren innerhalb eines partikulären Konstrukts recht hoch aufgrund der erzielbaren hohen Zelldichte bei noch nicht eintretender Kontaktinhibierung. Die Zelldichte läßt sich insofern herstellungstechnisch leicht optimieren. Zum zweiten sind die Zellen durch die umgebende Trägerstruktur gegen Scherbelastung gut geschützt. Zum dritten ist die Applikation sehr einfach, da eine Dispersion enthaltend die Konstrukte lediglich in eine Wunde eingebracht werden braucht, und zwar ohne unmittelbar vorherige Mischung mit einem Reagenz. Schließlich erfolgt das (ohnehin durch den hohen Pegel an Wachstumsfaktoren geförderte) Wachstum der Zellen dreidimensional und folglich ist die Bildungsrate von Gewebe beachtlich hoch gegenüber lediglich zweidimensionalen Gewebekonstrukten.

Im Rahmen dieser Beschreibung werden die folgenden Definitionen verwendet.

Der Ausdruck biologisch aktiv meint bezogen auf Zellen, daß zumindest 20%, vorzugsweise zumindest 50%, höchstvorzugsweise zumindest 80%, bezogen auf die Zellzahlen, der Zellen proliferationsfähig sind, gemessen beispielsweise mittels des MTS proliferation assay (CellTitre 96 AQueous One Solution Cell Proliferation Assay, Promega).

Ein partikuläres Konstrukt hat eine ausgeprägte dreidimensionale Erstreckung im Gegensatz zu flächigen Konstrukten, welche sich im wesentlichen in zwei Raumdimensionen erstrecken. Es handelt sich, mit anderen Worten ausgedrückt, um ein Partikel, dessen maximale Erstreckungen in den 3 Raumdimensionen sich nicht beachtlich unterscheiden. Faktoren zwischen den maximalen Erstreckungen in 2 beliebigen der 3 Raumdimensionen liegen unter 10, vorzugsweise unter 5, höchstvorzugsweise unter 2. Partikuläre Konstrukte haben insbesondere eine im wesentlichen sphärische Außenform (Faktoren unter 1,2). Partikuläre Konstrukte können massiv, hohl oder porös sein. Massiv meint hierbei eine Porosität (offene + geschlossene Porosität) von weniger als 5 Vol.-%, gemessen beispielsweise durch Neutronenkleinwinkelstreuung.

Eine räumliche Trägerstruktur bezeichnet ein Traggerüst aus einem festen Stoff, welches dem partikulären Konstrukt seine Gestalt gibt. Durch die Trägerstruktur sind die darin angeordneten Zellen fixiert und immobilisiert.

Biokompatibel sind Polymere, welche (human-)verträglich sind, also keine immunologischen Reaktionen eines (menschlichen) Körpers oder Vergiftungserscheinungen hervorrufen. Biokompatible Polymere sind weiterhin von einem (menschlichen) Organismus resorbierbar und/oder in Körperflüssigkeiten auflösbar. Dabei sind die biokompatiblen Polymere so ausgewählt, daß die Trägerstruktur eines in ein humanes Hautzellysat eingebrachten Konstrukts mit einem Trägerstrukturgewicht von 0,1 mg innerhalb einer Zeitspanne von 0,1 bis 20 Tage, vorzugsweise 1 bis 10 Tage, höchstvorzugsweise 2 bis 5 Tage, vollständig abgebaut bzw. aufgelöst ist.

Transplantationsmedizin meint insbesondere die Medizin des Hautersatzes durch künstliche Hautgewebe bildende Konstrukte. Hautersatz kann beispielsweise erforderlich werden bei Verbrennungswunden oder zum Schließen anderer Hautdefektwunden (beispielsweise angeborenen Hautdefekten und/oder Durchblutungsstörungen), wobei die Defektwunden auch im Zuge einer medizinisch notwendigen Exzission entstanden sein können. Die Transplantationsmedizin im Sinne der Erfindung umfaßt aber auch den Ersatz und/oder die Ergänzung anderer Gewebearten durch geeignete Zellen. Dies kann insbesondere zweckmäßig sein, wenn ein Gewebe Funktionsstörungen aufweist. Dann ist ein Ersatz oder eine Ergänzung beispielsweise mit Zellen möglich, welche diese Funktionsstörung nicht aufweisen. Der Ausdruck der Transplantationsmedizin umfaßt aber auch veterinärmedizinische Applikationen.

Der Ausdruck "ausgehärtet" umfaßt sowohl die Vernetzung von Polymermolekülen als auch die Trocknung. Trocknung ist die Solidifizierung von Polymerzusammensetzungen aus einer die Zusammensetzung enthaltenden Lösung oder Dispersion (wäßrig oder organisch), beispielsweise durch Austreibung des Lösungsmittels oder Entmischung der Zusammensetzung von dem Lösungsmittel.

Ein Vernetzungsgrad des Polymers ist durch den relativen Anteil an vernetzten Polymermolekülen zu der Gesamtzahl der Polymermoleküle (vernetzt + unvernetzt) gegeben.

Physiologisch aktive Wirkstoffe sind Stoffe, welche den Stoffwechsel beeinflussen, und zwar des Organismus und/oder der Zellen eines Konstrukts. Es versteht sich, daß solche Wirkstoffe in einer wirksamen Dosis eingesetzt werden.

Ein Konstrukt weist nicht kontaktinhibierte Zellen auf, wenn zumindest 20%, vorzugsweise zumindest 50%, höchstvorzugsweise zumindest 70%, idealerweise 80% bis 100%, der Zellen in dem Konstrukt nicht kontaktinhibiert sind.

Autologe Zellen sind Zellen aus einem Organismus, welche dann diesem Organismus wieder implantiert werden.

Die Aushärtung eines Polymers kann auf verschiedene Weisen erfolgen. Zum ersten kann das Polymer geliert und so solidifiziert werden, beispielsweise durch Temperaturerniedrigung gegenüber der Temperatur des flüssigen Polymers. Zum zweiten kann eine Solidifizierung durch chemische Reaktion erfolgen. Zum Dritten kann die Solidifizierung enzymatisch erfolgen. Für letzteres ist die Reaktion Fibrinogen/Thrombin ein Beispiel. Ein Aushärtungsreagenz ist eine Substanz oder eine Mischung von Substanzen, welche ein ungehärtetes Polymer auf chemischem und/oder enzymatischem Wege solidifiziert. Ein ungehärtetes Polymer ist ein flüssiges Polymer. Ein gehärtetes Polymer ist ein solidifiziertes Polymer.

Eine Dispergierlösung ist eine flüssige Phase, in welcher eine zweite, nicht oder nur schwer mischbare Flüssigkeit oder eine feste Substanz dispergiert werden kann. Dispersion meint hierbei, daß die eindispergierte Phase praktisch nicht aggregiert. Eine Dispergierlösung kann hierzu beispielsweise Emulgatoren, z.B. Lecithin oder Triton X100, enthalten.

Eine Zellsuspension besteht im wesentlichen aus vereinzelten Zellen in einer flüssigen Phase, beispielsweise in ungehärtetem Polymer und/oder in Medium.

Subkonfluenz liegt im Falle von Fibroblasten und/oder Keratinozyten typischerweise bei Zelldichten von unterhalb 1,5*10⁵ Zellen / cm², insbesondere von unterhalb 5*10⁴ Zellen / cm², vor.

Der Ausdruck des Eintropfens oder Vertropfens meint, daß oberhalb einer Dispergierlösung aus einer flüssigen Phase Tropfen gebildet werden, welche praktisch ohne weiteren Zerfall in kleinere Tropfen in die Dispergierlösung fallen und auch innerhalb dieser praktisch nicht desintegriert, sondern intakt bleibend lediglich in der Schwebe gehalten werden.

Im folgenden werden bevorzugte Ausführungsformen der Erfindung näher erläutert.

Die Trägerstruktur kann ein biokompatibles Polymer oder mehrere solcher Polymere aus der Gruppe bestehend "Fibrin, Collagen I, Hyaluronsäure, Collagen-Glycosaminglycan und Chondroitin-6-Sulfat" enthalten, insbesondere hieraus bestehen. Diese Polymere zeichnen sich durch ausgezeichnete Humanverträglichkeit und hinsichtlich der Resorptionskinetik gutes Verhalten (Resorption der Trägerstruktur eines Konstrukts in einer Hautwunde innerhalb von 1 bis 5 Tagen) aus. Zweckmäßigerweise weist das Polymer einen Vernetzungsgrad von weniger als 30%, vorzugsweise von weniger als 15%, auf.

Vorteilhaft kann es sein, wenn die Trägerstruktur einen physiologisch aktiven Wirkstoff oder mehrere solcher Wirkstoffe aus der Gruppe bestehend aus "therapeutische Wirkstoffe, Vitamine, Vitaminderivate, Wachstumsfaktoren, Glycocorticosteroide, Steroide, Antibiotika, antibakterielle Substanzen, antivirale Substanzen, Fungizide, Cytostatika, Tumorhemmer, Enzyme, Enzyminhibitoren, Proteine, Peptide, Minerale, Neurotransmitter, Lipoproteine, Glycoproteine, Immunomodulatoren, Immunoglobuline und Fragmente hiervon, Fettsäurederivate, Polysaccharide, antiinflamatorische Substanzen, Nucleinsäuren, Polynucleotide und Anästhetica" enthält. Mittels Anästhetika kann lokal eine Schmerzlinderung im Wundbereich erzielt werden. Andere Substanzen der Gruppe verhindern entzündliche Reaktionen. Wachstumsfaktoren fördern die Regeneration des neuen Gewebes zusätzlich. In jeden Fall ist es wesentlich, daß der Wirkstoff nicht die Proliferation des in dem Konstrukt eingesetzten Zelltyps hemmt oder gar Apoptose bewirkt.

Der humane Zelltyp ist ausgewählt aus der Gruppe "Fibroplasten, Fettzellen, Keratinocyten, Chondrocyten, Osteoplasten, Endothelzellen, Nervenzellen, Leberzellen, Pankreaszellen, Milzzellen, Nierenzellen und Muskelzellen", wobei vorzugsweise autologe Zellen eingesetzt werden. Fibroblasten, Keratinozyten und/oder Fettzellen sind insbesondere im Bereich der Hauttransplantation geeignet. Die weiteren Zelltypen eignen sich in Fällen, in welchen das entsprechende Gewebe eines Patienten Funktionsstörungen aufweist. Lediglich als Beispiel sei genannt die Implantation intakter Pankreaszellen, mittels welcher eine mangelnde Insulinproduktion wiederhergestellt werden kann. Hierbei kann auch mit autologen Zellen gearbeitet werden, welche beispielsweise gentechnisch gleichsam repariert worden sind.

Ein erfindungsgemäß verwendbares Konstrukt ist beispielsweise dadurch erhältlich, daß eine Suspension aus vereinzelten Zellen in einem biokompatiblen Polymeren, optional mit einem eine Aushärtung des Polymeren bewirkenden Reagenz versetzt, ungehärtete partikuläre Konstrukte bildend in eine Dispergierlösung hineindispergiert wird und diese Konstrukte in der Dispergierlösung gehärtet werden. Mit der Eintropftechnologie werden Konstrukte mit besonders gleichförmigen Dimensionen erhalten. Der Durchmesser erhaltener Konstrukte liegt typischerweise im Bereich von 0,05mm bis 5mm, vorzugsweise zwischen 0,1mm und 2mm, und ist im Rahmen der Eintropftechnologie wählbar. Die Streung im Durchmesser ist besonders klein. Es ist erreichbar, daß zumindest 30%, vorzugsweise zumindest 50%, höchstvorzugsweise zumindest 80%, der erhaltenen Konstrukte, bezogen auf die Anzahl, innerhalb eines Durchmesserbereiches von -50% bis +50%, vorzugsweise von -20% bis +20%, höchstvorzugsweise von -10% bis +10%, eines vorgegebenen Durchmesserwertes liegen.

Die Erfindung betrifft auch ein besonders vorteilhafterweise einsetzbares Konstrukt, welches dadurch erhältlich ist, daß eine Zellsuspension zusammen mit einem biokompatiblen Polymer in eine Dispergierlösung eingetropft und die entstehenden Tropfen in der Dispergierlösung gehärtet werden. Dabei wird das Polymer vorzugsweise unmittelbar vor der Einbringung in die Dispergierlösung mit einem Aushärtungsreagenz, vorzugsweise einem enzymatischen Aushärtungsreagenz, bspw. Thrombin, versetzt. Die Dispergierlösung ist nicht wäßrig und weist vorteilhafterweise zwei (flüssige) Phasen auf, wobei die Dichten der beiden Phasen so gewählt sind, daß die Konstrukte nicht weiter als bis zur Phasengrenze zwischen den beiden Phasen sedimentieren können. Hierdurch wird bewirkt, daß eingetropfte ungehärtete Konstrukte nicht bis zum Boden eines die Dispergierlösung enthaltenden Gefäßes absinken können, sondern jedenfalls im Bereich der Phasengrenze in der Schwebe gehalten werden. Hierdurch wird einerseits eine Desintegration der ungehärteten Konstrukte in der Dipergierlösung vermieden und andererseits kann beispielsweise ein Magnetrührer als Agitationselement verwendet werden, ohne daß das Agitationselement die Konstrukte mechanisch zu beschädigen vermag. Grundsätzlich kann auch noch mit einer dritten flüssigen Phase gearbeitet werden, welche dann zuoberst angeordnet ist und eine geringere Dichte als die Konstrukte, ungehärtet oder gehärtet, aufweist.

Nach der Härtung der Konstrukte ist es möglich, die Zellen vor der Transplantation in dem Konstrukt in fachüblicher Weise zu kultiviert. Dies gelingt gut, da erfindungsgemäße Konstrukte porös sind und beispielsweise Medium daher Zugang zum gesamten Konstruktvolumen hat. Die Kultivierung ist auch besonders unproblematisch, weil die Zellen mechanisch geschützt sind. Es können daher hocheffiziente Kultivierungstechniken eingesetzt werden (beispielsweise in gerührten und/oder begasten Bioreaktoren bzw. Säulen) mit der Folge, daß die Dauer der Herstellung eines autologen Konstrukts beachtlich reduziert ist. Zudem können Konstrukte mit verschiedenen Zelltypen, beispielsweise Fibroblasten und Keratinozyten, in einem einzigen Reaktor, getrennt lediglich durch eine Membran, unter gleichen Bedingungen kultiviert werden. Vorteilhafterweise wird eine Kultivierung nur solange durchgeführt, wie Subkonfluenz gewahrt ist. Hierdurch ist auch gewährleistet, daß eine Differenzierung der Zellen nicht in vitro, sondern in vivo erfolgt, was zur Rekonstruktion beispielsweise von Hautgewebe besonders vorteilhaft ist.

Die Erfindung lehrt auch ein Verfahren zur Herstellung eines biologisch aktiven partikulären Konstrukts, wobei eine Suspension aus einer Mehrzahl von vereinzelten humanen Zellen in einem biokompatiblen Polymer, ungehärtete partikuläre Konstrukte bildend, in eine Dispergierlösung eingetropft wird, wobei die ungehärteten partikulären Konstrukte in der Dispergierlösung gehärtet werden und wobei die gehärteten partikulären Konstrukte der Dispergierlösung entnommen werden. Hierbei werden (poröse) Vollkugeln erhalten in dem Sinne, daß die Zellen im Konstruktvolumen im wesentlichen gleichförmig verteilt sind. Vorzugsweise werden die ungehärteten partikulären Konstrukte durch Vertropfung aus einer Kapillaren gebildet und danach in die Dispergierlösung eingebracht.

Anisotrope Konstrukte lassen sich erzielen, wenn ungehärtete partikuläre Konstrukte dadurch erzeugt werden, daß eine Suspension enthaltend vereinzelte Zellen sowie ein biokompatibles Polymer einer Vertropfungseinrichtung zugeführt werden, wobei die Vertropfungseinrichtung eine zentrale Zellsuspensionskapillaröffnung und eine koaxial hierzu, hierum angeordnete Polymerkapillaröffnung aufweist, wobei die Suspension enthaltend vereinzelte Zellen sowie das Polymer gleichzeitig aus der Zellsuspensionskapillaröffnung sowie der Polymerkapillaröffnung zu ungehärteten partikulären Konstrukten vertropft und in die Dispergierlösung eingebracht werden, wobei die ungehärteten partikulären Konstrukte in der Dispergierlösung gehärtet werden und wobei die gehärteten partikulären Konstrukte der Dispergierlösung entnommen werden. Es werden dann Hohlkugeln in dem Sinne erhalten, daß das Polymer einen Kugelmantel bildet, in welchem die Zellen angeordnet sind, wobei die Zellen nicht in einer Polymermatrix eingebettet sind. Solche Konstrukte sind zellwachstumsmäßig vorteilhaft, da der Austausch von selbstgebildeten Wachstumsfaktoren verbessert ist.

Im Rahmen der Vertropfungstechnologie kann es sich empfehlen, daß beim Vertropfen ein Gasstrommantel erzeugt wird. Hierzu kann im Bereich einer Kapilarauslaßöffnung eine zur Kapillarauslaßöffnung koaxiale ringförmige Gasauslaßöffnung vorgesehen sein. Der Gasstrommantel fördert eine gleichförmige Tropfenbildung und kann (anstatt oder zusätzlich zu dem Volumenstrom aus der Kapallarauslaßöffnung) auch zur Steuerung der Tropfengröße herangezogen und variiert werden.

Eine hinsichtlich der Tropfenbildung und -ausbildung vorteilhafte Vertropfungstechnologie besteht darin, daß beim Vertropfen in einer Kapillaren rotationssymmetrische Schwingungen in dem Flüssigkeitsstrahl in der Kapilaren erzeugt und dem Flüssigkeitsstrahl überlagert werden, wobei die Wellenlänge der Schwingungen größer als der Umfang des Flüssigkeitsstrahls ohne Überlagerung von Schwingungen ist.

Ungehärtete und/oder gehärtete Konstrukte, ggf. nach Kultivierung, enthalten vorzugsweise 10⁴ bis 10⁶ Zellen je ml Konstruktvolumen.

Im folgenden wird die Erfindung anhand von Ausführungsbeispielen näher erläutert.

### Beispiel 1

V79 Firoblasten wurden in Kulturflaschen in Medium DMEM/F12 mit 3% FCS angezogen. Das Ablösen und Vereinzeln der Zellen erfolgte nach Entfernung des Mediums mit einer PBS/EDTA Lösung. Anschließend wurden die abgelösten Zellen in ein Zentrifugenröhrchen überführt und 5 min. bei 1000 rpm zentrifugiert. Der Überstand wurde verworfen und die Zellen in 200µl Medium DMEM/F12 mit HEPES und 3% FCS aufgenommen.

### Beispiel 2

Die in Beispiel 1 erhaltenen Zellen (Einsaatdichte: 10⁴ bis 10⁶ Zellen je ml) wurden wie folgt verkapselt. 1ml Fibrinogen-Lösung (TISSUCOL® -Kit 1,0 der Firma Immuno; 126-198mg Trockensubstanz enthalten 80-120mg Humanplasmaproteinfraktion mit 70-110mg Fibrinogen, 2-9mg Plasmafibronectin, 10-50 E [1E entspricht derjenigen Aktivität, die in 1ml frischem Normalplasma enthalten ist] Blutgerinnungsfaktor XIII und 0,02-0,08mg Plasminogen, Rest: Hilfstoffe NaCl, Natriumcitrat, Aprotin, Glycin, Heparin, Triton, Human Albumin) wurde nach Beipackzettel (Produktfassung: 01.06.1999) hergestellt. 200µl der resuspendierten Zellen aus Beispiel 1 wurden in der Fibrinogenlösung aufgenommen. und gut darin vermischt (Gesamtvolumen: 1,2ml). 1ml Thrombin L-Lösung (TISSUCOL® Kit der Firma Immuno, Produktfassung: 01.06.1999) wurde nach Beipackzettel hergestellt und mit 200µl sterilem Injektionswasser versetzt (Gesamtvolumen: 1,2ml). Beide Lösungen wurden in verschiedenen Kammern einer Doppelspritze mit unmittelbar benachbarten Austrittsdüsen der Kammern eingebracht. Durch Betätigung der beiden Spritzenkolben (gleiche Volumenströme aus den Kammern erzeugend) wurden Tropfen erzeugt, wobei sich beide Lösungen im Bereich der Austrittsdüsen vereinigten. Die Tropfen wurden eine Dispergierlösung fallen gelassen. Die Dispergierlösung bestand aus Fluorinert® Dielektrika FC40 der Firma 3M (gemäß 3M Datenblatt in der Fassung vom 03.01.1998 eine primäre Perfluorverbindung mit 12 Kohlenstoffatomen) sowie, darüber geschichtet, Miglyol® der Firma Hüls AG (Triglyceride von C8 bis C12 Fettsäuren). Die so erhaltene Diespergierlösung wurde vor dem Einsatz 20 min. bei 121°C autoklaviert. Die Dispergierlösung befand sich in einem Becherglas mit randgängigem Rührfisch auf einem Magnetrührer (200 - 500 rpm) und auf Raumtemperatur. Dann wurde 1h bei Raumtemperatur ausgehärtet unter Rühren. Danach erfolgte eine Separierung der erhaltenen Konstrukte von der Dispergierlösung gefolgt von dreimaligem Waschen mit Medium DMEM/F12. Anschließend wurde eine Kultivierung in einem 250ml Erlenmeyerkolben, enthaltend 50ml FC40, 75ml DMEM/F12 mit HEPES und 3% FCS, durchführt unter Rühren mit Magnetrührer. Erhalten wurden Konstrukte mit einem mittleren Durchmesser von ca. 1mm.

### Beispiel 3

Fibroblasten wurden in vorstehender Weise vereinzelt und einer Kollagen I Lösung zugegeben. Die Lösung wurde erhalten durch Lösung des Kollagens in Medium mittels HCl und anschließender Neutralisation mit NaCl. Die So erhaltene Zellsuspension wurde aus einer einfachen Spritze in die vorstehend beschriebene Dispergierlösung eingetropft. Nach 16h bei 37°C wurden im Wege des Gellierens solidifizierte Konstrukte erhalten und wie vorstehend beschrieben weiter behandelt.

## Patentansprüche

1. Biologisch aktives partikuläres Konstrukt auf Basis einer räumlichen Trägerstruktur aus zumindest einem ausgehärteten biokompatiblen Polymer, wobei in der Trägerstruktur eine Mehrzahl von Zellen zumindest eines Zelltyps eingebettet ist,
das erhalten wird, indem eine Suspension aus einer Mehrzahl von vereinzelten humanen Zellen in einem biokompatiblen Polymer, ungehärtete partikuläre Konstrukte bildend, in eine nicht wässrige Dispergierlösung eingetropft wird, wobei die ungehärteten partikulären Konstrukte in der Dispergierlösung gehärtet werden und wobei die gehärteten partikulären Konstrukte der Dispergierlösung entnommen werden.

2. Konstrukt nach Anspruch 1, wobei die Trägerstruktur ein biokompatibles Polymer oder mehrere solcher Polymere aus der Gruppe bestehend "Fibrin, Collagen I, Hyaluronsäure, Collagen-Glycosaminglycan und Chondroitin-6-Sulfat" enthält.

3. Konstrukt nach Anspruch 1 oder 2, wobei das Polymer einen Vernetzungsgrad von weniger als 30% aufweist.

4. Konstrukt nach einem der Ansprüche 1-3, wobei die Trägerstruktur einen physiologisch aktiven Wirkstoff oder mehrere solcher Wirkstoffe aus der Gruppe bestehend aus "therapeutische Wirkstoffe, Vitamine, Vitaminderivate, Wachstumsfaktoren, Glycocorticosteroide, Steroide, Antibiotika, antibakterielle Substanzen, antivirale Substanzen, Fungizide, Cytostatika, Tumorhemmer, Enzyme, Enzyminhibitoren, Proteine, Peptide, Minerale, Neurotransmitter, Lipoproteine, Glycoproteine, Immunomodulatoren, Immunoglobuline und Fragmente hiervon, Fettsäurederivate, Polysaccharide, antiinflamatorische Substanzen, Nucleinsäuren, Polynucleotide und Anästetica" enthält.

5. Konstrukt nach einem der Ansprüche 1-4, wobei der humane Zelltyp ausgewählt ist aus der Gruppe "Fibroplasten, Fettzellen, Keratinocyten, Chondrocyten, Osteoplasten, Endothelzellen, Nervenzellen, Leberzellen, Pancreaszellen, Milzzellen, Nierenzellen und Muskelzellen" und wobei vorzugsweise autologe Zellen eingesetzt werden.

6. Konstrukt nach einem der Ansprüche 1-5, dadurch erhältlich, daß eine Suspension aus vereinzelten Zellen in einem biokompatiblen Polymeren, optional mit einem eine Aushärtung des Polymeren bewirkenden Reagenz versetzt, ungehärtete partikuläre Konstrukte bildend in eine Dispergierlösung hineindispergiert wird und wobei diese Konstrukte in der Dispergierlösung gehärtet werden.

7. Konstrukt nach einem der Ansprüche 1-6, dadurch erhältlich, daß eine Zellsuspension zusammen mit einem biokompatiblen Polymer in eine Dispergierlösung eingetropft und die entstehenden Tropfen in der Dispergierlösung gehärtet werden.

8. Konstrukt nach einem der Ansprüche 6 oder 7, wobei das Polymer vorzugsweise unmittelbar vor der Einbringung in die Dispergierlösung mit einem Aushärtungsreagenz, vorzugsweise einem enzymatischen Aushärtungsreagenz, bspw. Thrombin, versetzt wird.

9. Konstrukt nach einem der Ansprüche 6-8, wobei die Dispergierlösung zwei Phasen aufweist, wobei die Dichten der beiden Phasen so gewählt sind, daß die Konstrukte nicht weiter als bis zur Phasengrenze zwischen den beiden Phasen sedimentieren können.

10. Konstrukt nach einem der Ansprüche 1-9, wobei die Zellen in dem Konstrukt kultiviert sind.

11. Konstrukt nach einem der Ansprüche 1-10, wobei die Zellen in dem Konstrukt subkonfluent sind.

12. Verfahren zur Herstellung eines biologisch aktiven partikulären Konstrukts, wobei eine Suspension aus einer Mehrzahl von vereinzelten humanen Zellen in einem biokompatiblen Polymer, ungehärtete partikuläre Konstrukte bildend, in eine nicht wässrige Dispergierlösung eingetropft wird, wobei die ungehärteten partikulären Konstrukte in der Dispergierlösung gehärtet werden und wobei die gehärteten partikulären Konstrukte der Dispergierlösung entnommen werden.

13. Verfahren nach Anspruch 12, wobei die ungehärteten partikulären Konstrukte durch Vertropfung aus einer Kapillaren gebildet und danach in die Dispergierlösung eingebracht werden.

14. Verfahren nach Anspruch 12, wobei ungehärtete partikuläre Konstrukte dadurch erzeugt werden, daß die Suspension enthaltend vereinzelte Zellen sowie das biokompatible Polymer einer Vertropfungseinrichtung zugeführt werden, wobei die Vertropfungseinrichtung eine zentrale Zellsuspensionskapillaröffnung und eine koaxial hierzu, hierum angeordnete Polymerkapillaröffnung aufweist, wobei die Suspension enthaltend vereinzelte Zellen sowie das Polymer gleichzeitig aus der Zellsuspensionskapillaröffnung sowie der Polymerkapillaröffnung zu ungehärteten partikulären Konstrukten vertropft und in die Dispergierlösung eingebracht werden, wobei die ungehärteten partikulären Konstrukte in der Dispergierlösung gehärtet werden und wobei die gehärteten partikulären Konstrukte der Dispergierlösung entnommen werden.

15. Verfahren nach Anspruch 13 oder 14, wobei beim Vertropfen ein Gasstrommantel erzeugt wird.

16. Verfahren nach einem der Ansprüche 13-15, wobei beim Vertropfen in einer Kapillaren rotationssymmetrische Schwingungen in dem Flüssigkeitsstrahl in der Kapillaren erzeugt und dem Flüssigkeitsstrahl überlagert werden, wobei die Wellenlänge der Schwingungen größer als der Umfang des Flüssigkeitsstrahls ohne Überlagerung von Schwingungen ist.

17. Verwendung eines biologisch aktiven partikulären Konstrukts nach einem der Ansprüche 1 bis 11 zur Herstellung eines Transplantats für die Transplantationschirurgie.

## Claims

1. Biologically active particulate construct based on a three-dimensional carrier structure made from at least one cured bio-compatible polymer, wherein a plurality of cells of at least one cell type is embedded into the carrier structure,
which is obtainable by dripping a suspension of a plurality of separated human cells in a bio-compatible polymer into a non-aqueous dispersion solution, wherein uncured particulate constructs are formed, wherein the uncured particulate constructs are cured in the dispersion solution, and wherein the cured particulate constructs are taken from the dispersion solution.

2. Construct according to claim 1, wherein the carrier structure comprises a bio-compatible polymer or several such polymers from the group consisting of "fibrin, collagen I, hyaluronic acid, collagen-glycosamineglycan, and chondroitin-6-sulfate".

3. Construct according to claim 1 or 2, wherein the polymer has a degree of cross-linking of less than 30%.

4. Construct according to one of the claims 1 - 3, wherein the carrier structure comprises a physiologically active pharmaceutical substance or several such pharmaceutical substances from the group consisting of "pharmaceutical substances for therapeutic purposes, vitamins, vitamin derivatives, growth factors, glycocorticosteroids, steroids, antibiotics, antibacterial substances, anti-viral substances, fungicides, cytostatika, tumor inhibitors, enzymes, enzyme inhibitors, proteins, peptides, minerals, neurotransmitters, lipoproteins, glycoproteins, immunmodulators, immunglobulins and fragments thereof, fatty acid derivatives, polysaccharides, anti-inflammatory substances, nucleic acids, polynucleotids, and anesthetica".

5. Construct according to one of the claims 1 - 4, wherein the human cell type is selected from the group of "fibroblasts, fat cells, keratinocytes, chondrocytes, osteoplasts, endothelial cells, nerve cells, liver cells, pancreas cells, spleen cells, kidney cells, and motoric cells", wherein preferably autologeous cells are used.

6. Construct according to one of the claims 1 - 5, obtainable by dispersing a suspension of separated cells in a bio-compatible polymer, optionally being supplied with an agent for curing the polymer, into a dispersion solution, wherein uncured particulate constructs are formed, and wherein these constructs are cured in the dispersion solution.

7. Construct according to one of the claims 1 - 6, obtainable by dripping a cell suspension together with a biocompatible polymer into a dispersion solution, and wherein the formed droplets are cured in the dispersion solution.

8. Construct according to claim 6 or 7, wherein the polymer is supplied with a curing agent, preferably an enzymatic curing agent, e.g. thrombin, preferably directly prior to bringing into the dispersion solution.

9. Construct according to one of the claims 6 - 8, wherein the dispersion solution comprises two phases, wherein the density of the two phases is selected such that the constructs do not sediment further than the phase boundary between the two phases.

10. Construct according to one of the claims 1 - 9, wherein the cells are cultivated in the construct.

11. Construct according to one of the claims 1 - 10, wherein the cells in the construct are sub-confluent.

12. Process for making a biologically active construct, wherein a suspension of a plurality of separated human cells in a bio-compatible polymer is dripped into a non-aqueous dispersion solution, wherein uncured particulate constructs are formed, wherein the uncured particulate constructs are cured in the dispersion solution, and wherein the cured particulate constructs are taken from the dispersion solution.

13. Process according to claim 12, wherein the uncured particulate constructs are formed by dripping from a capillary and, thereafter, bringing into the dispersion solution.

14. Process according to claim 12, wherein uncured particulate constructs are generated by introducing the suspension comprising separated cells and the biocompatible polymer into an dripping apparatus, wherein the dripping apparatus comprises a central capillary opening for the cell suspension and a polymer capillary opening for the polymer being arranged coaxial thereto and around, wherein the suspension comprising separated cells and the polymer are dripped simultaneously from the central capillary opening for the cell suspension and the polymer capillary opening for the polymer, forming uncured particulate constructs, and brought into the dispersion solution, wherein the uncured particulate constructs are cured in the dispersion solution, and wherein the cured particulate constructs are taken from the dispersion solution.

15. Process according to claim 13 or 14, wherein a gas stream curtain is formed when dripping.

16. Process according to one of the claims 13 - 15, wherein, when dripping, oscillations being of rotational symmetry are generated in the fluid stream within the capillary and superimposed to the fluid stream, wherein the wavelength of the oscillations is higher than the circumference of the fluid stream in absence of the oscillations.

17. Use of a biologically active particulate construct according to one of the claims 1 to 11 for making a transplant for transplatation surgery.

## Revendications

1. Structure particulaire biologiquement active sur la base d'une structure porteuse spatiale d'au moins un polymère biocompatible durci, dans la structure porteuse une multitude de cellules d'au moins un type de cellules étant encastrée,
ladite structure étant obtenue par ce qu'une suspension d'une multitude de cellules humaines individualisées dans un polymère biocompatible, en formant des structures particulaires non durcies, est égouttée dans une solution de dispersion non aqueuse, les structures particulaires non durcies étant durcies dans la solution de dispersion, et les structures particulaires durcies étant enlevées de la solution de dispersion.

2. Structure selon la revendications 1, la structure porteuse comprenant un polymère biocompatible ou plusieurs de tels polymères du groupe consistant en «fibrine, collagène I, acide hyaluronique, collagène-glycosaminoglycane et chondroitine-6-sulfate».

3. Structure selon la revendication 1 ou 2, le polymère ayant un degré de réticulation de moins de 30 %.

4. Structure selon une des revendications 1 à 3, la structure porteuse comprenant un agent physiologiquement actif ou plusieurs de tels agents du groupe consistant en «agents thérapeutiques, vitamines, dérivés de vitamines, facteurs de croissance, glyco-corticostéroïdes, stéroïdes, antibiotiques, substances antibactérielles, substances antivirales, fungicides, cytostatiques, inhibiteurs de la tumeur, enzymes, inhibiteurs enzymatiques, protéines, peptides, minéraux, neurotransmetteurs, lipoprotéines, glycoprotéines, immunomodulateurs, immunoglobulines et fragments de celles-ci, dérivés de l'acide gras, polysaccharides, substances antiinflammatoires, acides nucléiques, polynucléotides et anesthésiques».

5. Structure selon une des revendications 1 à 4, le type de cellules humaines étant choisi du groupe consistant en «fibroblastes, cellules adipeuses, kératinocytes, chondrocytes, ostéoplastes, cellules de l'endothélium, cellules nerveuses, cellules du foie, cellules du pancréas, cellules de la rate, cellules du rein et cellules musculaires», et de préférence des cellules autologues étant utilisées.

6. Structure selon une des revendications 1 à 5, ladite structure étant obtenue par ce qu'une suspension de cellules individualisées dans un polymère biocompatible, en option en combinaison avec un réactif provoquant le durcissement du polymère, en formant des structures particulaires non durcies, est dispersée dans une solution de dispersion, et lesdites structures sont durcies dans la solution de dispersion.

7. Structure selon une des revendications 1 à 6, ladite structure étant obtenue par ce qu'une suspension de cellules est égouttée en commun avec un polymère biocompatible dans une solution de dispersion, et les gouttes formées sont durcies dans la solution de dispersion.

8. Structure selon une des revendications 6 ou 7, ledit polymère étant réagi de préférence immédiatement avant l'introduction dans la solution de dispersion avec un réactif de durcissement, de préférence un réactif enzymatique de durcissement, p.ex. thrombine.

9. Structure selon une des revendications 6 à 8, ladite solution de dispersion comprenant deux phases, les densités des deux phases étant choisies de telle manière que les structures ne puissent sédimenter que jusqu'à la frontière entre des deux phases.

10. Structure selon une des revendications 1 à 9, lesdites cellules étant cultivées dans la structure.

11. Structure selon une des revendications 1 à 10, lesdites cellules étant subconfluentes dans la structure.

12. Procédé pour la production d'une structure particulaire biologiquement active, une suspension d'une multitude de cellules humaines individualisées dans un polymère biocompatible, en formant des structures particulaires non durcies, étant égouttée dans une solution de dispersion non aqueuse, les structures particulaires non durcies étant durcies dans la solution de dispersion, et les structures particulaires durcies étant enlevées de la solution de dispersion.

13. Procédé selon la revendication 12, lesdites structures particulaires non durcies étant formées par égouttage d'un tube capillaire et puis étant introduites dans la solution de dispersion.

14. Procédé selon la revendication 12, des structures particulaires non durcies étant produites par ce que la suspension comprenant des cellules individualisées et le polymère biocompatible sont amenés à un égouttoir, cet égouttoir comprenant une ouverture centrale du tube capillaire de la suspension des cellules et une ouverture du tube capillaire du polymère agencée coaxialement autour de celle-ci, ladite suspension comprenant des cellules individualisées et le polymère s'égouttant simultanément de l'ouverture du tube capillaire de la suspension des cellules et de l'ouverture du tube capillaire du polymère en formant des structures particulaires non durcies et étant introduits dans la solution de dispersion, les structures particulaires non durcies étant durcies dans la solution de dispersion et les structures particulaires durcies étant enlevées de la solution de dispersion.

15. Procédé selon la revendication 13 ou 14, lors dudit égouttage une enveloppe de courant de gaz étant générée.

16. Procédé selon la revendication 13 à 15, lors dudit égouttage dans un tube capillaire, des vibrations à symétrie de révolution étant générées dans le jet de liquide dans le tube capillaire et étant superposées au jet de liquide, les longueurs d'onde des vibrations étant plus grandes que le périmètre du jet de liquide sans une superposition de vibrations.

17. Utilisation d'une structure particulaire biologiquement active selon une des revendications 1 à 11 pour la production d'un transplant pour la chirurgie de transplantation.
